# EUROPEAN PATENT APPLICATION

(11) **EP 3 895 739 A1**
(43) Date of publication of application: **20.10.2021**
(21) Application number: 21168263.8
(22) Date of filing: 14.04.2021
(51) Int. Cl.: A61L 2/20, A61L 9/12, B60H 3/00, A61L 101/10

(54) **SANITIZING SYSTEM FOR A VEHICLE**

(30) Priority: 16.04.2020 IT 202000008077
(71) Applicant: Texa SpA, 31050 Monastier di Treviso (TV) (IT)
(72) Inventor: VIANELLO, Bruno, 31050 MONASTIER DI TREVISO (TV) (IT)
(74) Representative: Burchielli, Riccardo

(57) **Abstract**

Described is a sanitizing system for a vehicle, comprising a sanitizing device (30; 31), placed inside the vehicle (10) and designed to dispense chemical agents and/or sanitizing gas into the vehicle (10), and a remote control device (20), installed on the vehicle (10) and designed for controlling the sanitizing device (30; 31) and, in particular, the dispensing of chemical agents and/or sanitizing gas into the vehicle (10). The sanitizing system is connected to a control and/or diagnosis unit (40) of the vehicle (10), which is connected through an OBD socket to a control unit of an air conditioning system of the vehicle (10).

## Description

This invention relates generally to a sanitizing system for a vehicle.

In particular, the invention relates to a sanitizing system directly connected to the remote control of the vehicle.

Vehicles which are used every day, regardless of their type, are highly exposed to bacterial contamination and viruses such as, for example, the current case of COVID-19, more commonly known as 'Coronavirus'.

In particular, the vehicles can be used for a wide variety of activities and jobs, just think of passenger vehicles such as, for example, buses, taxis and ambulances.

A phenomenon of contamination which often occurs, particularly in these types of vehicles, is cross-contamination, that is, the direct or indirect passage of microbes and pathogens through a source, such as, in this case, the passenger compartment of the vehicle, through the likely contact of the hands of several people.

Maintaining a clean environment free from contamination, including viruses, is currently one of the most sought-after objectives in the automobile sector; reducing the infections from bacteria and viruses means not only ensuring hygiene, but also preventing the spread and proliferation of diseases which could affect a multitude of people.

The sanitizing systems currently comprise the introduction of chemical agents or gases, such as ozone, into the vehicle through a nebulizing system which is able sterilize its internal surfaces.

This operation is not normally carried out with people inside the passenger compartment, as the sanitizing substances are often harmful to humans in the event of inhalation, swallowing or contact with the mucous membranes or eyes.

Moreover, with the sanitizing systems currently used in the automobile sector, it is not possible to have direct control on the consumption of the sanitizing product used, with respect to the volume of the vehicle to be sanitized.

One of the main problems with the sanitizing systems currently used is due to the fact that not all the surfaces of a vehicle are directly reachable by the sanitizing substances; for example, hoses, fittings, valves, filters, doors, etc. are not effectively subjected to the sanitizing process when it is taking place in the passenger compartment of the vehicle.

The above considerably limits the effectiveness of the sanitizing operation; in fact, as there can be no personnel inside the vehicle during the sanitizing, the process is consequently limited, making it difficult for the sanitizing gas to reach areas of the vehicle which are not easily accessible, such as the ducts of the air conditioning system.

Over recent years there has been a significant increase in the occurrence of respiratory diseases due to poor management and lack of maintenance of the air conditioning systems of automobiles.

Air conditioning systems not only control the thermal conditions of an environment, but also filter all airborne particles. A direct consequence of this function is the accumulation of chemical and microbiological contaminants which, by saturating the system, paradoxically transform it into a source of pollution and contamination.

In addition to the obvious health and hygiene implications, this leads to higher running costs for the system due to the increased resistance encountered by the air, the reduced efficiency of the heat exchange coils, the acceleration of the natural corrosion process of the dehumidification system and of the coils, etc.

For this reason, without this important intervention in the ducts, the sanitizing substances are only useful inside the passenger compartment. For the above-mentioned reasons, it is necessary to use a sanitizing system which allows and favours the disinfection not only of the passenger compartment of the vehicle, but also, and above all, those surfaces which are not easily and directly reachable, guaranteeing a control of the sanitizing process in its entirety.

An aim of the invention is therefore to develop a sanitizing system for vehicles which guarantees an increase in the efficiency of the sanitizing operations also for all those surfaces of the vehicle which cannot be easily reached or for which the sanitizing needs to reach them through precise sequential commands of the air conditioning system of the vehicle. Another aim of the invention is to develop a sanitizing system for vehicles which is easier and faster to use than the prior art methods.

A further aim of the invention is to develop a sanitation system for vehicles which is more efficient than the prior art.

These and other aims are achieved by a sanitizing system for a vehicle according to the appended claim 1; other detailed features of the sanitizing system are set out in the dependent claims.

Advantageously, unlike the solutions described and proposed so far, the sanitizing system according to the invention not only allows the passenger compartment of the vehicle to be sanitized, but also acts effectively on all areas of the vehicle which cannot be reached, such as for example all ducts of the air conditioning system of the vehicle, vents and filters.

The above-mentioned aims and the advantages are described in more detail below, relative to a preferred embodiment of the sanitizing system, according to the invention, provided by way of illustration and example, but without limiting the scope of the invention, and in the accompanying drawings, also provided by way of a non-limiting example, wherein:
- Figure 1 is a front and external view, with respect to the vehicle, of a remote control device forming part of the sanitizing system for vehicles according to the invention;
- Figure 2 is a perspective and internal view, with respect to the vehicle, of the remote control device of Figure 1 according to the invention;
- Figure 3 is a perspective and internal view, with respect to the vehicle, of a second embodiment of the control device of Figure 1 according to the invention;
- Figure 4 is a schematic view of the sanitizing system according to the invention.

With reference to the above-mentioned drawings, the reference numeral 10 generally indicates the vehicle on which the sanitizing system according to the invention is installed.

The reference numeral 20 indicates a remote control device installed on the vehicle 10, for controlling the sanitizing process, for example by the user.

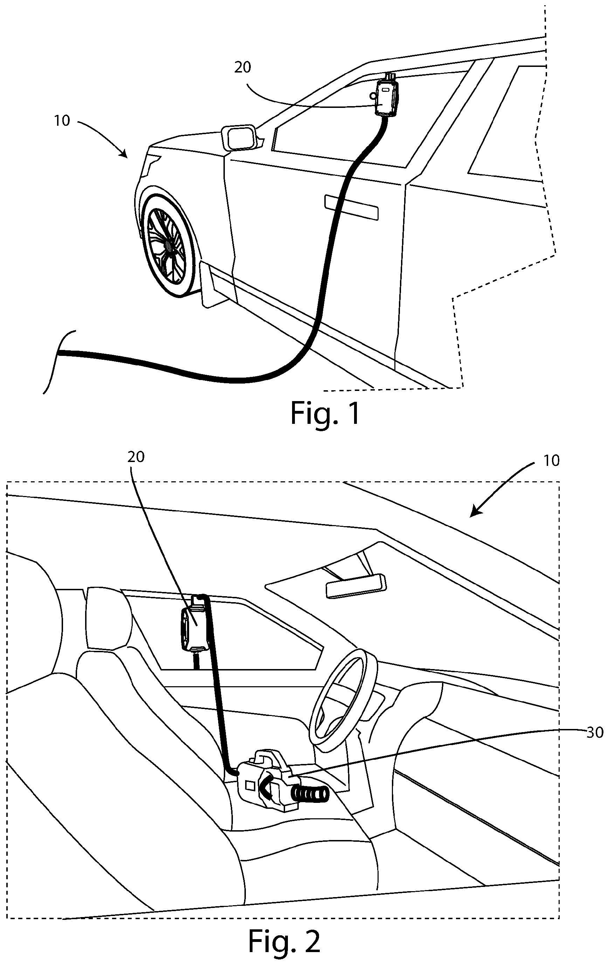

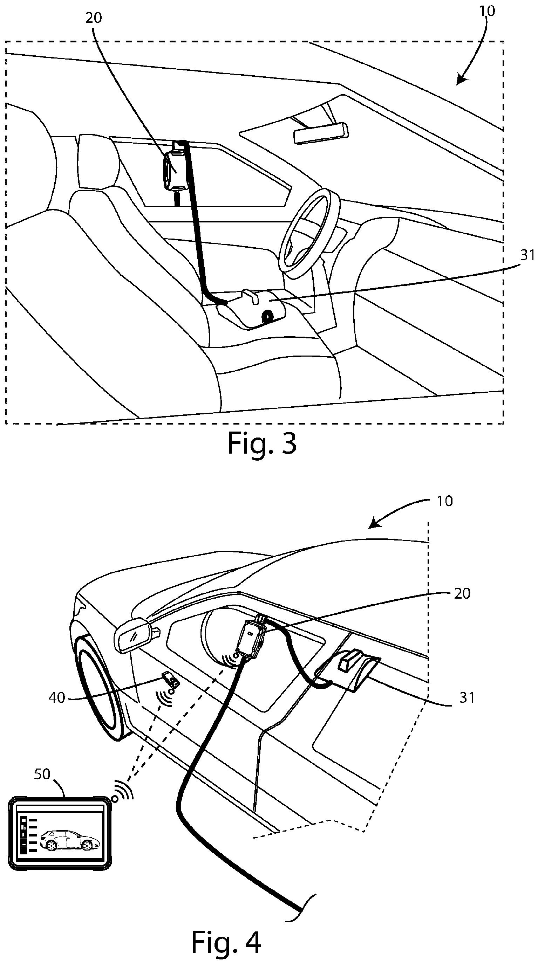

According to preferred embodiments, the above-mentioned remote control device 20 is installed on the window of the vehicle 10 and is connected, through a first cable placed outside the vehicle, to a power supply source and, through a second cable placed inside the vehicle, to a sanitizing device 30, as can be seen in detail in the accompanying Figures 2 and 3.

The sanitizing device 30 is advantageously placed inside the vehicle and has, in preferred embodiments, a dispensing opening designed to facilitate the diffusion of the sanitizing gas inside the passenger compartment of the vehicle 10.

The above-mentioned sanitizing device 30 may be of different types and/or embodiments, such as the one shown in Figure 3, wherein a particular embodiment of the device 30 is shown, according to which the above-mentioned device, denoted with reference numeral 31, has a fan for dispensing the sanitizing gas, instead of the above-mentioned dispensing opening.

Advantageously, the above-mentioned sanitizing device 30, 31 is designed to dispense inside the vehicle 10, through the command received from the remote control device 20, various types of chemical agents or gas depending on the sanitizing operation being carried out.

Advantageously, one of the gases most commonly used by the sanitizing system, according to the invention, is Ozone; currently, Ozone is a gas whose peculiarities are well known with regard to its disinfecting and, therefore, sanitizing power and, in addition to being recognised as usable in the circumstances described above, it has also been recognised as usable by the legislations in force (in fact, the Italian Ministry of Health, with protocol no. 24482 of 31 July 1996, recognised the sanitizing system with Ozone as a valid method for sterilizing contaminated environments, with a series of scientific validations).

As can be seen in Figure 4, according to preferred embodiments, the sanitizing system, according to the invention, is connected to a control and/or diagnosis unit 40 of the vehicle 10 to be sanitized, in particular a VCU (Vehicle Control Unit).

Advantageously, the above-mentioned control and/or diagnosis unit 40 is in turn connected, through the OBD socket of the vehicle 10 to the air conditioning control unit of the vehicle. Also advantageously, the sanitizing system according to the invention may allow, for example, an operator located outside the vehicle 10, and by using a suitable software 50, to manage the control of the air conditioning system of the vehicle 10 from the outside; this avoids the presence of an operator inside the passenger compartment of the vehicle 10 to use the control commands of the air conditioning system, which are usually present on the dashboard of the vehicle.

In detail, by having an external control on the air conditioning system, the entire sanitizing operation will be constantly guided and will allow the access of the sanitizing product also in the surfaces otherwise not reachable; the above is only possible by activating from the outside, with the system according to the invention, for example, the fans of the air conditioning system, the air recirculation, the opening and closing of the various aeration and suction openings, etc., in order to condition and direct the movement of the air and therefore of the sanitizing gas inside the vehicle.

Moreover, again advantageously, the above-mentioned software 50 will be able to control the consumption of the sanitizing product, by means of utilization curves advantageously set on the basis of the volume of the passenger compartment of the vehicle 10 and as a function of the control mode of the air conditioning system.

According to preferred embodiments, all the communication operations of the sanitizing system according to the invention and, in particular, the controls provided by the above-mentioned software 50 are performed through wireless connections and, in particular, through Bluetooth®. Moreover, as can be seen in figure 5, the sanitizing system can be controlled and managed through an electronic device, such as a tablet, PC, smartphone, etc.

By virtue of the foregoing, it can be understood how the sanitizing system for vehicles, according to the invention, achieves the above-mentioned aims and advantages.

Lastly, it is clear that numerous other variants might be made to the sanitizing system according to the invention, without thereby departing from the scope of protection of the invention expressed in the accompanying claims, whilst it is also clear that in the practical actuation of the invention, the materials, the shapes and the dimensions of the illustrated details can be of any type and can be replaced, according to requirements, by other technically equivalent elements.

## Claims

1. Sanitizing system for a vehicle (10) comprising:
- a sanitizing device (30; 31), placed inside a vehicle (10) and configured to deliver in said vehicle (10) chemical agents and/or sanitizing gas;
- a remote control device (20) configured to control said sanitizing device (30; 31), so as to control the supply of said chemical agents and/or said sanitizing gas in the vehicle (10),
**characterized in that** said remote control device (20) is connected to a control and/or diagnosis unit (40) of said vehicle (10), wherein said control or diagnosis unit (40) is connected, through an OBD socket, to a control unit of a vehicle air conditioning system (10).

2. Sanitizing system as claimed in claim 1, **characterized in that** said remote control device (20) is configured to control, by means of said control unit of said vehicle air conditioning system (10), the fans, the air recirculation ducts, the openings and closings and intake vents of said air conditioning system, in order to direct the flow of said sanitizing gas inside the vehicle (10).

3. Sanitizing system as claimed in at least one of the preceding claims, **characterized in that** it comprises a software (50) configured to manage the consumption of said chemical agents and/or sanitizing gas through utilization curves, wherein said utilization curves are set on the basis of the volume of a passenger compartment of said vehicle (10) and on the basis of the data given by said remote control device (20) of the air conditioning system.

4. Sanitizing system as claimed in claim 1, **characterized in that** said remote control device (20) is installed on a window of said vehicle (10).

5. Sanitizing system as claimed in at least one of the preceding claims, **characterized in that** said remote control device (20) is connected, through a first cable, to a power source and, through a second cable which is located inside said vehicle (10), to said sanitizing device (30; 31).

6. Sanitizing system as claimed in at least one of the preceding claims, **characterized in that** said chemical agents and/or sanitizing gas supplied by said sanitizing device (30; 31) include Ozone.

7. Sanitizing system as claimed in at least one of the preceding claims, **characterized in that** said software (50) communicates with said sanitizing system via wireless connection and in particular via Bluetooth®.

8. Sanitizing system as claimed in at least one of the preceding claims, **characterized in that** said system is controlled and managed through a mobile or portable electronic device, such as a tablet, PC, smartphone, etc.
